# EUROPÄISCHE PATENTSCHRIFT

(11) **EP 2 981 275 B1**
(45) Veröffentlichungstag und Bekanntmachung des Hinweises auf die Patenterteilung: **09.01.2019**
(21) Anmeldenummer: 14715256.5
(22) Anmeldetag: 31.03.2014
(51) Int. Cl.: A61K 36/09, A61P 1/04

(54) **LUTSCHTABLETTE ZUR BEHANDLUNG VON HALSSCHMERZ, HEISERKEIT UND ASSOZIIERTEM TROCKENEN HUSTEN, SOWIE ENTZÜNDLICHEN ERKRANKUNGEN DES MUND UND RACHENRAUMS**
LOZENGE FOR THE TREATMENT OF THROAT PAIN, HOARSENESS AND ASSOCIATED DRY COUGH, AS WELL AS INFLAMMATORY DISEASES OF THE MOUTH AND PHARYNX
PRODUIT EN PASTILLE POUR LE TRAITEMENT DU MAL DE GORGE, L'ENROUEMENT ET LA TOUX SÈCHE ASSOCIÉE, ET DES MALADIES INFLAMMATOIRES DE LA CAVITÉ BUCCALE ET DE LA GORGE

(30) Priorität: 02.04.2013 EP 13161902
(43) Veröffentlichungstag der Anmeldung: 10.02.2016
(73) Patentinhaber: Sanofi-Aventis Deutschland GmbH, 65929 Frankfurt am Main (DE)
(72) Erfinder: BONI, Julia, 55216 Ingelheim am Rhein (DE); PLOHMANN, Bernd, 55216 Ingelheim am Rhein (DE); SAUERLAND, Sandra, 55216 Ingelheim am Rhein (DE)
(74) Vertreter: Catillon, Marie
(86) Internationale Anmeldenummer: PCT/EP2014/056437
(87) Internationale Veröffentlichungsnummer: WO 2014/161811

(56) Entgegenhaltungen:
- EP-A1- 2 361 623
- DATABASE WPI Week 201105 Thomson Scientific, London, GB; AN 2011-A54615 XP002708379, & JP 2011 001321 A (WAKAN SEIYAKU KENKYUSHO KK) 6. Januar 2011 (2011-01-06)
- DATABASE TCM [Online] sipo; 26. Januar 2005 (2005-01-26), Lv Wenxiao, Zhong Weidong: "A kind of laryngopharynx health tea and its preparation method/A kind of new chinese medicinal composition and its preparation of medical tea, which can used for the prevention and treatment of acute/chronic pharyngolaryngitis, stomatitis and upper respiratory infection", XP002708380, Database accession no. CN1569192

## Beschreibung

Die vorliegende Erfindung betrifft eine Pharmazeutische Darreichungsform, enthaltend eine Kombination aus (a) wenigstens einem adstringierenden Wirkstoff und (b) wenigstens einer Schleimdroge sowie deren Verwendung zur Vorbeugung oder Behandlung von entzündlichen Erkrankungen des Mund- und Rachenraums sowie zur Behandlung von schmerzhaften Irritationen der Schleimhäute im Mund- und Rachenbereich und damit verbundenem Reizhusten.

### HINTERGRUND DER ERFINDUNG

Die vorliegende Erfindung betrifft eine pharmazeutisch Zusammensetzung in Form einer zum Lutschen geeigneten Darreichungsform, wobei die Zusammensetzung eine Kombination mindestens eines adstringierenden Wirkstoffs mit mindestens einer zweiten Schleimstoffdroge bzw. deren Extrakt enthält, zur Behandlung insbesondere von Irritationen und entzündlichen Erkrankungen des Mund- und Rachenraums, Mundtrockenheit und Husten.

Die Mundschleimhaut dient in besonderem Maße dem Schutz des darunterliegenden Gewebes und dient als natürliche Barriere vor mechanischen Verletzungen (Läsionen) und gegen das Eindringen von Mikroorganismen und toxischen Substanzen. Sie befeuchtet die Mundhöhle und benetzt Nahrungsmittel um deren Verdauung zu ermöglichen.

Der Hals- / Rachenraum ist ausgekleidet mit einer Schleimhaut, deren wesentlicher Bestandteil das Epithel ist. Das Epithel ist die Schnittstelle zwischen menschlichem Körper und aufgenommenen Substanzen und bildet eine komplexe physikochemische Barriere, welche, vervollständigt durch den mukoziliären Apparat die erste Abwehr gegen Pathogene bildet. Schleim bedeckt das das Epithel und bietet einen zusätzlichen Schutz der Schleimhaut in dem es eine semipermeable Barriere bildet, die den Austausch von Nährstoffen, Wasser und Gasen ermöglicht, pathogene Keime jedoch abhält. Der Schleim ist ein komplex zusammengesetztes, viskoelastisches Gel welches kontinuierlich von intraepithelialen Zellen und Schleimdrüsen sezerniert wird. Hauptbestandteil des Schleims sind Muzine, große und stark geladene Glykoproteine die durch Quervernetzung das strukturelle Gebäude der Schleimhautbarriere bilden.

Wichtig für die mukoziliäre Clearance ist die Anzahl der Zilien, ihrer Struktur und Aktivität, sowie deren koordinierten Bewegung. Optimale Funktion ist bei 37 °C und einer absoluten Feuchte von 44 mg/dm³ (entspricht 100 % relativer Feuchte) gewährleistet. Kälte, trockene oder überhitzte Raumluft, sowie Alkohol oder Nicotin beinträchtigen die normale Schleimhautfunktion und können zu Schleimhautirritation, Heiserkeit, Halsschmerz und trockenem Husten führen. Unter solchen Umständen ist eine bakterielle Besiedelung begünstig und pulmonäre Infektionen können die Folge sein.

Oftmals treten entzündliche Erkrankungen des Mund- und Rachenraums auch als Begleiterscheinung von Erkältungserkrankungen und grippalen Infekten auf. Häufig sind diese mit einer für den Patienten unangenehmen Schmerzsymptomatik und Schluckbeschwerden verbunden. Auch Entzündungen der Mundhöhle wie z.B. Gingivitis, Stomatitis oder Mundschleimhautläsionen weisen eine für den betroffenen Patienten unangenehme Symptomatik auf.

Typischerweise erfolgt die Behandlung entzündlicher Erkrankungen des Mund- und Rachenraums durch topische Therapie beispielsweise in Form von Mund- und Rachenspülungen, Sprays oder Lutschtabletten. In schwerwiegenden Fällen erfolgt zusätzlich eine systemische Therapie, z.B. durch Gabe von Antibiotika.

Insbesondere hat sich 1-Hexadecylpyridiniumchlorid (internationaler Freiname: "Cetylpyridiniumchlorid (CPC)) zur topischen Behandlung von entzündlichen Erkrankungen des Mund- und Rachenraums bewährt. Hierbei handelt es sich um eine quaternäre Ammoniumverbindung mit bakterizider und fungizider Wirkung, welche unter anderem in Lutschtabletten zur Anwendung kommt. Nachteilig bei diesem Wirkstoff ist die Tatsache, dass bei hoher Dosierung und übermäßigem Verzehr Magen-Darm-Beschwerden, Atemnot sowie eine vermehrte Methämoglobinbildung, insbesondere bei Kindern, auftreten kann.

Desweiteren hat sich als topisches Antiseptikum bzw. Desinfektionsmittel für die Mund-, Hals- und Rachenschleimhaut auch 1,3-Bis(2-ethylhexyl)-hexahydr-5-methyl-5-pyridinamin (internationaler Freiname: "Hexetidin") bewährt, welches beispielsweise als Spray oder in Form von Spül- bzw. Gurgellösungen angewendet wird. Bei längerer oder starker Dosierung kann es auch hier zu Magen-Darm-Beschwerden und darüber hinaus zu Geschmacksirritationen kommen.

Als weitere Wirkstoffe zur topischen Behandlung entzündlicher Prozesse des Mund- und Rachenraums haben sich adstringierende Wirkstoffe, insbesondere Kaliumaluminiumsulfat-Decahydrat (internationale Freiname: Alaun), bewährt. Hierbei handelt es sich um ein Salz, welches traditionell zur Behandlung von Läsionen im Mund, Gingivitis oder Schleimhautschwellungen und -blutungen zur Anwendung kommt. Durch den adstringierenden Effekt verringert es zudem die Besiedelung durch Keime und kann durch seine abschwellende Wirkungen Schmerzen mildern. Nachteilig ist das durch den adstringierenden Effekt hervorgerufenes Gefühl von Mundtrockenheit.

Desweiteren kommen Wirkstoffe natürlichen Ursprungs zum Einsatz, so z.B. sogenannte Schleimdrogen oder deren Extrakte, wie z.B. Isländisch Moos (Lichen Islandicus). Diese Schleimdrogen enthalten in hoher Konzentration Poylsaccharide, welche in Verbindung mit Wasser Schleime bilden. Angewendet bei entzündlichen Erkrankungen des Mund- und Rachenraums in Form von z.B. Lutschtabletten oder Sprays, formen sie eine schützende Schicht und befeuchten die Schleimhaut. Diese zusätzliche Schleimschicht schützt die empfindlichen Mechano- und Chemorezeptoren in der Schleimhaut vor äußeren Reizen und verringert dadurch Irritationen wie z.B. Kratzen, Heiserkeit und Hustenreiz. Üblicherweise werden diese Wirkstoffe in Form von Monopräparaten eingesetzt, erzielen aber nicht immer den gewünschten Erfolg.

Der vorliegenden Erfindung liegt daher die Aufgabe zugrunde, eine pharmazeutische Zubereitung in Form einer festen Darreichungsform zur topischen Behandlung von entzündlichen Erkrankungen des Mund- und Rachenraums bereitzustellen, welche die zuvor geschilderten Nachteile des Standes der Technik zumindest weitgehend vermeidet oder abschwächen soll.

Die Anmelderin hat nun überraschenderweise herausgefunden, dass das zuvor geschilderte Problem dadurch gelöst wird, dass man eine pharmazeutische Zubereitung in Form einer festen Dosierung von mindestens einem adstringierenden Wirkstoff (z.B. Alaun) mit mindestens einer Schleimdroge und/oder deren Extrakt (z.B. Isländisch Moos) formuliert. Insbesondere geeignet ist eine solche Zusammensetzung in Form von Lutschtabletten, hergestellt durch Kompression von Pulvermischungen oder auf Basis von Hartkaramellen, welche bei schmerzhaften bzw. schmerzbegleitenden entzündlichen Erkrankungen des Mund- und Rachenraums zur topischen Anwendung gelangen können.

### ERFINDUNGSGEGENSTAND

Gegenstand der vorliegenden Erfindung sind Pharmazeutische Darreichungsform enthaltend eine Kombination aus (a) wenigstens einem adstringierenden Wirkstoff und (b) wenigstens einer Schleimdroge.

Die vorgenannte Kombination eignet sich insbesondere zur Vorbeugung und Behandlung, speziell zur symptomatischen Vorbeugung oder Behandlung von entzündlichen Erkrankungen des Mund- und Rachenraums. Zudem eignet sich die vorgenannte Kombination zur vorübergehenden alleinigen oder unterstützenden Behandlung der zuvor genannten Erkrankungen.

Folglich betrifft die vorliegende Erfindung weiterhin die Verwendung der erfindungsgemäßen Pharmazeutischen Darreichungsform zur Vorbeugung oder Behandlung von entzündlichen Erkrankungen des Mund- und Rachenraums.

Ebenso betrifft die vorliegende Erfindung die Verwendung der erfindungsgemäßen Pharmazeutischen Darreichungsform zur Behandlung von schmerzhaften Irritationen der Schleimhäute im Mund- und Rachenbereich und damit verbundenem Reizhusten.

Ebenso betrifft die vorliegende Erfindung die erfindungsgemäße Kombination aus (a) wenigstens einem adstringierenden Wirkstoff und (b) wenigstens einer Schleimdroge, sowie deren Verwendung bei der Vorbeugung oder Behandlung von entzündlichen Erkrankungen des Mund- und Rachenraums und/oder deren Verwendung bei der Behandlung von schmerzhaften Irritation der Schleimhäute im Mund- und Rachenbereich und damit verbundenem Reizhusten.

Durch die Verwendung der erfindungsgemäßen Kombination auf Basis von mindestens einem adstringierenden Wirkstoff und mindestens einer Schleimdroge resultiert bei hoher Wirksamkeit gleichzeitig eine gute Verträglichkeit einhergehend mit einer weitestgehenden Verhinderung von Nebenwirkungen.

Das Wirkprinzip der erfindungsgemäßen Zusammensetzung basiert auf rein physikalischen bzw. physikochemischen Prozessen. Das grundlegende Prinzip der vorliegenden Erfindung beruht dabei auf einer gezielten Kombination von adstringierenden Wirkstoffen einerseits und Wirkstoffkomponenten auf Basis von Schleimdrogen andererseits, wobei die Erfinder herausgefunden haben, dass sich die Wirksamkeit von adstringierenden Wirkstoffen einerseits und Schleimdrogen andererseits, in Bezug auf die zuvor angeführten Erkrankungen, durch die erfindungsgemäße Kombination in nicht zu erwartender Weise erhöht.

### BESCHREIBUNG

Der Begriff "Kombination" wie hierin verwendet beschreibt wenigstens zwei Wirkstoffe, ausgewählt unter (a) adstringierenden Wirkstoffen und (b) Schleimdrogen, die in einem zur gemeinsamen Einnahme geeigneten Verhältnis zueinander vorliegen.

Der Begriff "Schleimdroge" wie hierin verwendet umfasst die Schleimdroge, deren Bestandteile und deren Extrakte. So umfasst beispielsweise der Begriff "Isländisch Moos" wie hierin verwendet, Isländisch Moos, dessen Bestandteile und dessen Extrakte.

Insbesondere eignen sich als erfindungsgemäß verwendete Schleimdrogen Isländisch Moos (Lichen islandicus), Malve (Malva sylvestris), Spitzwegerich (Plantago lanceolata), Eibisch (Althaea offizinalis) und Huflattich (Tussilago farfara), sowie deren Gemische. Bevorzugt wird Isländisch Moos, speziell in Form eines Extrakts, verwendet.

Der Begriff "adstringierenden Wirkstoff" wie hierin verwendet umfasst Wirkstoffe, die beim Auftreffen auf Haut oder Schleimhaut durch Eiweißfällung austrocknend, blutstillend und entzündungshemmend wirken. Insbesondere eignen sich als adstringierender Wirkstoff Alaun, Tannin, Policresulen und Silbernitrat oder deren Gemische. Bevorzugt wird Alaun verwendet.

Eine spezielle Ausführungsform der vorliegenden Erfindung betrifft eine Pharmazeutische Darreichungsform nach einem der vorhergehenden Ansprüche bestehend aus (a) wenigstens einem adstringierender Wirkstoff; (b) wenigstens einer Schleimdroge und (c) wenigstens einem weiteren Hilfsstoff.

Der Begriff "Pharmazeutische Darreichungsform" wie hierin verwendet umfasst insbesondere übliche feste pharmazeutische Darreichungsformen, wie Lutschtabletten, beispielsweise hergestellt durch Pulverkompression oder auf Hartkaramellenbasis.

Geeignete Hilfsstoffe sind insbesondere Tablettierhilfen und Füllstoffe, beispielsweise Isomalt, Fructose- und/oder Glucosesirup, Siliciumdioxid, Talcum, Magnesiumstearat, Macrogol, Polydextrose oder Xanthangummi, Süssungsmittel, beispielsweise Zuckeraustauschstoffe, insbesondere Isomalt, Sorbitol, Polydextrose, Maltit oder Isomaltit, Süßstoffe, beispielsweise Aspartam, Acesulfam, Cyclamat, Saccharin oder Xylitol, Aromen, Farbstoffe und/oder Stabilisatoren, beispielsweise Weinsäure oder Zitronensäure.

Der Gewichtsanteil der verwendeten Hilfsstoffe liegt üblicherweise in einem Bereich von 50 bis 99.5 Gew.-%, bevorzugt von 75 bis 99 Gew.-%, besonders bevorzugt von 85 bis 95 Gew.-%, jeweils bezogen auf das Gesamtgewicht der Pharmazeutischen Darreichungsform.

In einer speziellen Ausführungsform der erfindungsgemäßen Pharmazeutische Darreichungsform, wird Isländisch Moos als Schleimdroge verwendet. Isländisch Moos wird hierbei bevorzugt als Extrakt verwendet.

Geeignete Extraktevon Isländisch Moos werden beispielsweise durch wässrige, alkoholische oder wässrig-alkoholische Extraktion und gegebenenfalls anschließende Trocknung erhalten. Diese Extrakte bestehen üblicherweise hauptsächlich aus dem schleimformenden Poylsacchariden Lichenin und Isolichenin. Durch den erhöhten Gehalt an Schleimstoffen wird die therapeutische Wirkung gegenüber der reinen Droge verbessert. Neben den Schleimstoffen enthält der Extrakt Flechtensäuren die einen leichten antimikrobiellen Effekt aufweisen. Im Vergleich zu Droge ist die Verarbeitung der Extrakte vereinfacht.

In einer speziellen Ausführungsform der erfindungsgemäßen Pharmazeutische Darreichungsform, wird Malve als Schleimdroge verwendet. Malve wird hierbei bevorzugt als Extrakt verwendet.

Geeignete Extrakte von Malve werden beispielsweise durch wässrige, alkoholische oder wässrig-alkoholische Extraktion und gegebenenfalls anschließende Trocknung erhalten. Diese Extrakte bestehen üblicherweise größtenteils aus Schleimen. Diese setzen sich aus den quervernetzten Einzelbausteinen Glucose, Arabinose, Rhamnose und Glactose zusammen. Der Gehalt im Extrakt ist um ein vielfaches höher gegenüber der reinen Droge, wodurch der therapeutische Effekt verbessert wird.

Die erfindungsgemäßen Pharmazeutische Darreichungsform enthalten die Schleimdroge üblicherweise in einer Menge von 1 mg bis 500 mg, bevorzugt von 10 mg bis 250 mg, besonders bevorzugt von 50 mg bis 200 mg .

Der Gewichtsanteil der verwendeten Schleimdroge liegt üblicherweise in einem Bereich von 1 bis 50 Gew.-%, bevorzugt von 2 bis 25 Gew.-%, besonders bevorzugt von 5 bis 10 Gew.-%, jeweils bezogen auf das Gesamtgewicht der Pharmazeutischen Darreichungsform

In einer speziellen Ausführungsform der erfindungsgemäßen Pharmazeutische Darreichungsform, wird Alaun als adstringierende Wirkstoff verwendet.

In einer weiteren speziellen Ausführungsform der erfindungsgemäßen Pharmazeutische Darreichungsform, wird Tannin als adstringierende Wirkstoff verwendet.

Die erfindungsgemäßen Pharmazeutische Darreichungsform enthalten den adstringierenden Wirkstoff üblicherweise in einer Menge von 0.1 mg bis 50 mg, bevorzugt von 0.5 mg bis 20 mg besonders bevorzugt von 1 mg bis 10 mg .

Der Gewichtsanteil des verwendeten adstringierenden Wirkstoffs liegt üblicherweise in einem Bereich von 0.01 bis 5 Gew.-%, bevorzugt von 0.05 bis 2 Gew.-%, besonders bevorzugt von 0.1 bis 1 Gew.-%, jeweils bezogen auf das Gesamtgewicht der Pharmazeutischen Darreichungsform.

Eine bevorzugte Ausführungsform der vorliegenden Erfindung betrifft die Kombination von Isländisch Moos als Schleimdroge und Alaun als adstringierendem Wirkstoff.

Isländisch Moos wird hierbei üblicherweise in einer Menge von 1 mg bis 500 mg verwendet, bevorzugt in einer Menge von 10 mg bis 250 mg, besonders bevorzugt in einer Menge von 50 mg bis 200 mg.

Alaun wird hierbei üblicherweise in einer Menge von 0.1 mg bis 20 mg verwendet, bevorzugt in einer Menge von 1 mg bis 15 mg , besonders bevorzugt in einer Menge von 3 bis 10 mg . Das Gewichtsverhältnis von Isländisch Moos zu Alaun liegt dabei übleicherweise im Bereich von 5 : 1 bis 50 : 1, bevorzugt von 10 : 1 bis 25 : 1, besonders bevorzugt von 15 : 1 bis 20 : 1.

Der Gewichtsanteil von Isländisch Moos und Alaun liegt üblicherweise in einem Bereich von 0.5 bis 50 Gew.-%, bevorzugt von 1 bis 25 Gew.-%, besonders bevorzugt von 5 bis 15 Gew.-%, jeweils bezogen auf das Gesamtgewicht der Pharmazeutischen Darreichungsform

Die erfindungsgemäße pharmazeutische Darreichungsform wird bevorzugt als Lutschtablette bereitgestellt. Geeignete Lutschtabletten können beispielsweise durch Pulverkompression oder auf Hartkaramellenbasis hergestellt werden.

Das Gesamtgewicht der Pharmazeutischen Darreichungsform liegt dabei üblicherweise in einem Bereich von 500 mg bis 5000 mg, bevorzugt von 750 mg bis 4000 mg, besonders bevorzugt von 1000 mg bis 3000 mg.

Die erfindungsgemäße pharmazeutische Darreichungsform wird üblicherweise bis zu 20 mal täglich, bevorzugt bis zu 12 mal, besonders bevorzugt bis zu 8 mal täglich verabreicht.

Die erfindungsgemäße Kombination eignet sich zur Vorbeugung oder Behandlung von entzündlichen Erkrankungen des Mund- und Rachenraums. Dementsprechend eignet sich auch die erfindungsgemäße Pharmazeutische Darreichungsform für diese Verwendung.

Die erfindungsgemäße Kombination eignet sich insbesondere zur Vorbeugung oder Behandlung von schmerzbegleiteten bzw. schmerzhaften entzündlichen Erkrankungen des Mund- und Rachenraums. Dementsprechend eignet sich auch die erfindungsgemäße Pharmazeutische Darreichungsform für diese Verwendung.

Die erfindungsgemäße Kombination eignet sich insbesondere auch zur symptomatischen Behandlung von entzündlichen Erkrankungen des Mund- und Rachenraums. Dementsprechend eignet sich auch die erfindungsgemäße Pharmazeutische Darreichungsform für diese Verwendung.

Die erfindungsgemäße Kombination eignet sich insbesondere auch zur Behandlung der Entzündung und/oder der Schmerzen bei entzündlichen Erkrankungen des Mund- und Rachenraums. Dementsprechend eignet sich auch die erfindungsgemäße Pharmazeutische Darreichungsform für diese Verwendung.

Die erfindungsgemäße Kombination eignet sich insbesondere auch zur Behandlung von Reizhusten, insbesondere bei entzündlichen Erkrankungen des Mund- und Rachenraums. Dementsprechend eignet sich auch die erfindungsgemäße Pharmazeutische Darreichungsform für diese Verwendung.

Die erfindungsgemäße Kombination eignet sich insbesondere auch zur Behandlung von Schluckbeschwerden, insbesondere bei entzündlichen Erkrankungen des Mund- und Rachenraums. Dementsprechend eignet sich auch die erfindungsgemäße Pharmazeutische Darreichungsform für diese Verwendung.

Die erfindungsgemäße Kombination eignet sich insbesondere auch zum Wiedererlangen der durch Heiserkeit und Schmerzen eingeschränkten Stimme, insbesondere bei entzündlichen Erkrankungen des Mund- und Rachenraums. Dementsprechend eignet sich auch die erfindungsgemäße Pharmazeutische Darreichungsform für diese Verwendung.

Die erfindungsgemäße Kombination eignet sich insbesondere zur topischen Verabreichung der Wirkstoffe. Dementsprechend eignet sich auch die erfindungsgemäße Pharmazeutische Darreichungsform für diese Verwendung.

Die erfindungsgemäße Kombination eignet sich insbesondere auch zur Behandlung von schmerzhaften Irritationen der Schleimhäute im Mund- und Rachenbereich und damit verbundenem Reizhusten. Dementsprechend eignet sich auch die erfindungsgemäße Pharmazeutische Darreichungsform für diese Verwendung.

Die erfindungsgemäße Kombination hilft die gereizte und/oder entzündete Mund- und Rachenschleimhaut zu beruhigen und lindert den Halsschmerz.

Die erfindungsgemäße Kombination hilft bei der Regeneration der Schleimhäute bei erkältungsbedingten Symptomen.

Die erfindungsgemäße Kombination hilft bei der Behandlung von Halsschmerzen

Die erfindungsgemäße Kombination hilft die Symptome von Halsentzündungen und - reizungen, wie trockener, juckender, kratzender Hals, zu lindern.

Die erfindungsgemäße Kombination hilft den Hals gegen weitere Reizung zu schützen.

Die erfindungsgemäße Kombination wirkt als Schutzfilm der gereizten Schleimhaut gegen bakterielle Besiedelung.

Die erfindungsgemäße Kombination beruhigt die Mund- und Rachenschleimhaut und hilft so den Kreislauf von Halsbeschwerden und trockenem Husten zu durchbrechen.

Die erfindungsgemäße Kombination hilft die Symptome von trockenem Husten zu verringern und lindert den Hustenreiz.

Die erfindungsgemäße Kombination hilft den Hals zu beruhigen und zu befeuchten und lindert somit den Husten.

Die erfindungsgemäße Kombination hilft bei Schluckbeschwerden.

Die erfindungsgemäße Kombination hilft bei Heiserkeit.

Ein weiterer speziell Gegenstand der vorliegenden Erfindung betrifft die Kombination aus (a) wenigstens einem adstringierenden Wirkstoff und (b) wenigstens einer Schleimdroge zur Verwendung bei der Vorbeugung oder Behandlung von entzündlichen Erkrankungen des Mund- und Rachenraums.

Ein weiterer spezieller Gegenstand der vorliegenden Erfindung betrifft Kombination aus (a) wenigstens einem adstringierenden Wirkstoff und (b) wenigstens einer Schleimdroge zur Verwendung bei der Behandlung von schmerzhaften Irritationen der Schleimhäute im Mund- und Rachenbereich und damit verbundenem Reizhusten

### BEISPIELE

### 1. Herstellungsbeispiele

Herstellung erfindungsgemäßer pharmazeutischer Zubereitungen in Form von Lutschtabletten auf der Basis eines adstringierenden Wirkstoffs in Kombination mit unterschiedlichen Schleimdrogen.

Verschiedene Zusammensetzungen werden in Form von Lutschtabletten auf Basis von Pulverkompression oder Hartkaramellenbasis hergestellt. Für die komprimierten Lutschtabletten können vorzugsweise Zuckeraustauschstoffe, insbesondere Isomalt, Sorbitol oder Polydextrose, für die Hartkaramellenbasis Fructose- und/oder Glucosesirup, als zuckerfreie Variante Zuckeraustauschstoffe, insbesondere Zuckeralkohole, vorzugsweise Maltit oder Isomaltit eingesetzt. Variable Mengen an Süßstoffen (z.B. Aspartam, Acesulfam, Cyclamat, Saccharin oder Xylitol) können zugesetzt werden. Weitere Hilfs- und Wirkstoffe werden in die Matrix eingearbeitet und die Lutschtabletten in an sich bekannter Herstellweise gefertigt.

Im Falle der komprimierten Pulvermischungen werden die Hilfsstoffe entsprechend der Rezeptur gewogen und mit der Matrix gemischt. Im Falle von flüssigen Hilfs- oder Wirkstoffe können diese über einen weiteren Granulierschritt zugegeben werden. Anschließend werden Pulver bzw. Granulat zu Lutschtabletten verpresst. Die Hilfs- oder Wirkstoffe können wahlweise ein einer oder mehreren Schichten gemeinsam oder getrennt verpresst werden (1-, 2- oder 3-Schichttabletten).

Im Falle von Hartkaramellen werden die Rohstoffe entsprechend der Rezeptur gemischt und in die erhitzte Hartkaramellenbasis eingemischt. Hitzeempfindliche Hilfsstoffe und/oder Wirkstoffe, insbesondere
- Schleimdrogen, vorzugsweise Isländisch Moos
- Adstringierenden Wirkstoffe, vorzugsweise Alaun
- gegebenenfalls Aromen
- gegebenenfalls Farbstoffe
- gegebenenfalls Stabilisatoren, insbesondere Weinsäure oder Zitronensäure
werden über einen separaten Schritt kurz vor Endmischungung und Lutschtablettenformung zugegeben.

Auf diese Arten werden beispielsweise zuckerfreie Lutschtabletten mit unterschiedlichen Mengen an adstringierenden Wirkstoffen und Schleimdrogen hergestellt, wie in den nachfolgenden Tabellen dargestellt:

**Tabelle 1: 1-Schicht-Lutschtablette hergestellt über Pulverkompression mit 5 mg Alaun und 80 mg Isländisch Moos Extrakt**

| Rohstoffe | Menge pro Lutschtablette [mg] |
|---|---|
| Isomalt | 1200 |
| Siliciumdioxid | 8 |
| Zitronenaroma | 30 |
| Menthol | 2 |
| Talcum | 50 |
| Macrogol | 25 |
| Alaun | 5 |
| Isländisch Moos Extrakt (Droge/Extrakt 1:1.5-2.5) | 80 |
| gesamt | 1400 |

**Tabelle 2: 1-Schicht-Lutschtablette hergestellt über Pulverkompression mit 1 mg Tannin und 150 mg Malven Extrakt**

| Rohstoffe | Menge pro Lutschtablette [mg] |
|---|---|
| Polydextrose | 1100 |
| Siliciumdioxid | 10 |
| Erdbeeraroma | 30 |
| Menthol | 4 |
| Magnesiumstearat | 25 |
| Sucralose | 5 |
| Tannin | 1 |
| Malven Extrakt (Droge/Extrakt 4:1) | 150 |
| gesamt | 1325 |

**Tabelle 3: 2-Schicht-Lutschtablette hergestellt über Pulverkompression mit 5 mg Alaun und 80 mg Isländisch Moos Extrakt**

| Rohstoffe | Menge pro Lutschtablette - Schicht 1 [mg] | Menge pro Lutschtablette - Schicht 1 [mg] |
|---|---|---|
| Sorbitol | 590 | 490 |
| Siliciumdioxid | 3 | 3 |
| Erdbeeraroma | 15 | 15 |
| Eucalyptusaroma | 2 | 2 |
| Talcum | 25 | 25 |
| Macrogol | 10 | 10 |
| Xanthangummi | - | 25 |
| Alaun | - | 5 |
| Isländisch Moos Extrakt (Droge/Extrakt 1:1.5-2.5) | - | 80 |
| gesamt | 1300 | |

**Tabelle 4: Lutschtablette auf Hartkaramellenbasis mit 5 mg Alaun und 80 mg Isländisch Moos Extrakt**

| Rohstoffe | Menge pro Lutschtablette [mg] |
|---|---|
| Isomalt | 2500 |
| Pfirsicharoma | 15 |
| Menthol | 0.5 |
| Zitronensäure | 35 |
| Sucralose | 1.5 |
| Alaun | 5 |
| Isländisch Moos Flüssigextrakt (Droge/Extrakt 1:1.5-2.5) | 80 |
| Wasser | 53 |
| gesamt | 2690 |

### 2. Anwendungsbeispiele

30 Patienten mit einer schmerzhaften Irritation der Schleimhäute im Mund- und Rachenbereich und teilweise damit verbundenem Reizhusten wurden mit der in den vorangegangenen Herstellbeispielen beschriebenen Lutschtabletten ohne zusätzliche systemische Therapie therapiert. 10 Patienten wurden mit der in Tabelle 4 beschriebenen Zubereitung therapiert, wobei über einen Zeitraum von drei Tagen bis zu sechs Tabletten verabreicht wurden (Gruppe A). 10 Weitere wurden mit einem handelsüblichen Präparat nur mit Alaun (9 mg pro Lutschtablette) über drei Tage mit bis zu sechs Tabletten therapiert (Gruppe B). Weiteren 10 Patienten wurde ein handelsübliches Monopräparat mit Isländischen Moos (80 mg Isländisch Moos Extrakt pro Lutschtablette) nach ähnlichem Darreichungsschema verabreicht (Gruppe C).

Während sich unter Anwendung der erfindungsgemäßen Darreichungsform eine Linderung der gesamten durch die Entzündungen der Mund- und Rachenschleimhaut verursachten Symptome einstellte, zeigte sich unter Präparaten der Gruppe B und C nur eine Linderung einzelner Beschwerden.

Bei dem nichterfindungsgemäßen Präparat der Gruppe B zeigte sich eine Verbesserung der Entzündungs- und Schmerzsymptome der Schleimhaut. Kaum bis keine Verbesserung zeigte sich in Gruppe B bei den Symptomen der Heiserkeit und Mundtrockenheit und des damit verbundenen Reizhustens.

Unter Therapie mit dem nichterfindungsgemäßen Präparat der Gruppe C zeigte sich eine Verbesserung vor allem des durch den mit Entzündungen der Mund- und Rachenschleimhaut assoziierten Reizhustens, der Mundtrockenheit, sowie der Schluckbeschwerden.

Die Untersuchungen zeigen einen deutlich gesteigerten Therapieerfolg durch Behandlung mit der erfindungsgemäßen Darreichungsform. Aufgrund der Kombination von adstringierendem Wirkstoff und Schleimdroge trat bei den mit der erfindungsgemäßen Zusammensetzung behandelten Patienten bereits nach kurzer Therapiedauer eine deutliche Linderung des Hustenreizes, sowie der Entzündungs- der Schmerzsymptomatik auf. Eine rasche Linderung von Schluckbeschwerden wurde durch die Anwendung erreicht und die durch Heiserkeit und Schmerzen eingeschränkte Stimme rasch wiedererlangt. Zudem empfanden die Patienten das Lutschgefühl der erfindungsgemäßen Zusammensetzung als sehr angenehm. In der zuvor geschilderten Verbesserung der Symptomatik liegt ein entscheidender Vorteil der erfindungsgemäßen Zusammensetzung gegenüber nichterfindungsgemäßen Monopräparaten.

## Patentansprüche

1. Pharmazeutische Darreichungsform enthaltend eine Kombination aus (a) wenigstens einem adstringierenden Wirkstoff und (b) wenigstens einer Schleimdroge, worin die Schleimdroge ausgewählt ist unter Isländisch Moos, Malve, Spitzwegerich, Eibisch und Huflattich und worin der adstringierende Wirkstoff ausgewählt ist unter Alaun, Tannin, Policresulen und Silbernitrat.

2. Pharmazeutische Darreichungsform nach Anspruch 1, bestehend aus (a) wenigstens einem adstringierender Wirkstoff; (b) wenigstens einer Schleimdroge und (c) wenigstens einem weiteren Hilfsstoff.

3. Pharmazeutische Darreichungsform nach einem der vorhergehenden Ansprüche, worin die Schleimdroge Isländisch Moos ist.

4. Pharmazeutische Darreichungsform nach einem der vorhergehenden Ansprüche, enthaltend die Schleimdroge in einer Menge von 1 mg bis 500 mg.

5. Pharmazeutische Darreichungsform einem der vorhergehenden Ansprüche, worin der adstringierende Wirkstoff Alaun ist.

6. Pharmazeutische Darreichungsform nach einem der vorhergehenden Ansprüche, enthaltend den adstringierende Wirkstoff in einer Menge von 0.1 mg bis 50 mg.

7. Pharmazeutische Darreichungsform nach einem der vorhergehenden Ansprüche, bereitgestellt als Lutschtabletten.

8. Kombination aus (a) wenigstens einem adstringierenden Wirkstoff und (b) wenigstens einer Schleimdroge gemäß einem der Ansprüche 1 bis 7 zur Verwendung bei der Vorbeugung oder Behandlung von entzündlichen Erkrankungen des Mund- und Rachenraums.

9. Kombination aus (a) wenigstens einem adstringierenden Wirkstoff und (b) wenigstens einer Schleimdroge gemäß einem der Ansprüche 1 bis 7 zur Verwendung bei der Behandlung von schmerzhaften Irritation der Schleimhäute im Mund- und Rachenbereich und damit verbundenem Reizhusten.

## Claims

1. Pharmaceutical dosage form comprising a combination of (a) at least one astringent active substance and (b) at least one mucilaginous drug, wherein the mucilaginous drug is selected from Icelandic moss, mallow, lance leaf plantain, marshmallow and coltsfoot, and wherein the astringent active substance is selected from alum, tannin, policresulen and silver nitrate.

2. Pharmaceutical dosage form according to Claim 1, consisting of (a) at least one astringent active substance, (b) at least one mucilaginous drug and (c) at least one further adjuvant.

3. Pharmaceutical dosage form according to any of the preceding claims, wherein the mucilaginous drug is Icelandic moss.

4. Pharmaceutical dosage form according to any of the preceding claims, comprising the mucilaginous drug in an amount of 1 mg to 500 mg.

5. Pharmaceutical dosage form according to any of the preceding claims, wherein the astringent active substance is alum.

6. Pharmaceutical dosage form according to any of the preceding claims, comprising the astringent active substance in an amount of 0.1 mg to 50 mg.

7. Pharmaceutical dosage form according to any of the preceding claims, prepared as lozenges.

8. Combination of (a) at least one astringent active substance and (b) at least one mucilaginous drug according to any of Claims 1 to 7 for use in the prevention or treatment of inflammatory diseases of the mouth and the pharynx.

9. Combination of (a) at least one astringent active substance and (b) at least one mucilaginous drug according to any of Claims 1 to 7 for use in the treatment of painful irritation of the mucous membranes in the mouth and the pharynx and an associated dry cough.

## Revendications

1. Forme galénique pharmaceutique contenant une combinaison de (a) au moins un principe actif astringent et de (b) au moins une substance mucilagineuse, dans laquelle la substance mucilagineuse est choisie parmi la mousse d'Islande, la mauve, le plantain lancéolé, la guimauve et le pas-d'âne et dans laquelle le principe actif astringent est choisi parmi l'alun, le tannin, le polycrésulène et le nitrate d'argent.

2. Forme galénique pharmaceutique selon la revendication 1, constituée par (a) au moins un principe actif astringent ; (b) au moins une substance mucilagineuse et (c) au moins un autre adjuvant.

3. Forme galénique pharmaceutique selon l'une quelconque des revendications précédentes, dans laquelle la substance mucilagineuse est la mousse d'Islande.

4. Forme galénique pharmaceutique selon l'une quelconque des revendications précédentes, contenant la substance mucilagineuse en une quantité de 1 mg à 500 mg.

5. Forme galénique pharmaceutique selon l'une quelconque des revendications précédentes, dans laquelle le principe actif astringent est l'alun.

6. Forme galénique pharmaceutique selon l'une quelconque des revendications précédentes, contenant le principe actif astringent en une quantité de 0,1 mg à 50 mg.

7. Forme galénique pharmaceutique selon l'une quelconque des revendications précédentes, préparée sous forme de comprimés à sucer.

8. Combinaison de (a) au moins un principe actif astringent et (b) au moins une substance mucilagineuse selon l'une quelconque des revendications 1 à 7 pour l'utilisation lors de la prévention ou du traitement de maladies inflammatoires de la cavité pharyngobuccale.

9. Combinaison de (a) au moins un principe actif astringent et (b) au moins une substance mucilagineuse selon l'une quelconque des revendications 1 à 7 pour l'utilisation lors du traitement de l'irritation douloureuse des muqueuses au niveau de la bouche ou de la gorge et des toux sèches qui y sont associées.
